(19) Europäisches Patentamt
European Patent Office
Office européen des brevets

(11) EP 2 725 358 A1

# (12) EUROPEAN PATENT APPLICATION

(43) Date of publication:
**30.04.2014 Bulletin 2014/18**

(51) Int Cl.:
***G01N 33/532*** *(2006.01)* ***G01N 33/543*** *(2006.01)*
***A61K 47/48*** *(2006.01)*

(21) Application number: **12189516.3**

(22) Date of filing: **23.10.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Miltenyi Biotec GmbH**
**51429 Bergisch Gladbach (DE)**

(72) Inventors:
- **Dose, Christian, Dr.**
  **51429 Bergisch Gladbach (DE)**
- **Brieden, Jennifer**
  **51429 Bergisch Gladbach (DE)**

(74) Representative: **Kisters, Michael Marcus**
**Patentanwalt**
**Miltenyi Biotec GmbH**
**Corporate Legal Department**
**Friedrich-Ebert Strasse 68**
**51429 Bergisch Gladbach (DE)**

## (54) Release system for cell-antibody-substrate conjugates containing a polyethylene glycol spacer unit

(57) The invention is directed to a releasable conjugate comprising a biotinylated ligand (ligand$_1$) having a biotin moiety, a ligand moiety, and a biotin-binding molecule (bbm) bound to the biotin moiety of the biotinylated ligand wherin the biotin moiety and the ligand moiety of the biotinylated ligand are separated by a spacer group consisting of polyethylene glycol according to the general formula I

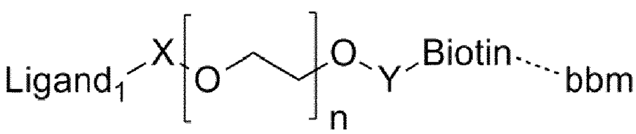

I

with n = 1 - 500, and X, Y = same or different, substituted or unsubstituted alkyl groups with 1 to 20 carbon atoms.

Furthermore, the invention relates to a method for cleaving the releasable conjugate by providing biotin, streptavidin, or derivates thereof in a sufficient concentration to displace the biotin-binding molecule (bbm) from the biotin moiety of the biotinylated ligand.

EP 2 725 358 A1

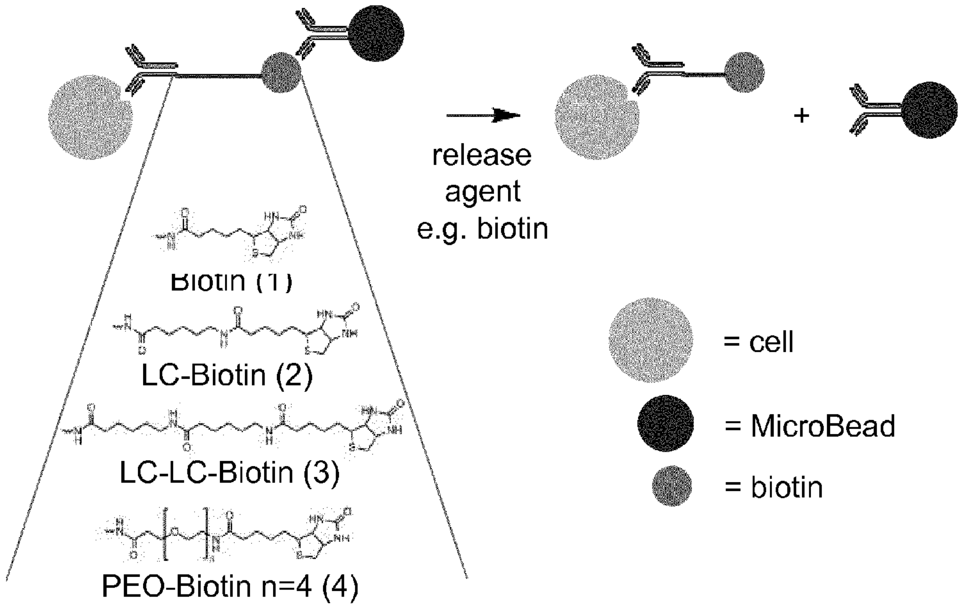
release
agent
e.g. biotin
Biotin (1)
LC-Biotin (2)
LC-LC-Biotin (3)
PEO-Biotin n=4 (4)
+
= cell
= MicroBead
= biotin

Fig.1

## Description

### Field of invention

The present invention relates to a releasable conjugate comprising a biotinylated ligand and a biotin-binding molecule bound to the biotin moiety of the biotinylated ligand, wherein the biotin moiety and the ligand are separated by a spacer group; and a method to selectively dissociate the releasable conjugate.

### Background of the invention

The ability of biotin to bind streptavidin, avidin, and other biotin-binding molecules has been exploited for several decades, because of the high affinity, specificity, and broad applicability of this system.

Besides many other applications, biotin is also used to label living cells via biotinylated antibodies for cell separation applications. These methodologies usually encompass two consecutively performed steps. First, a selective binding of the target cells by biotinylated antibodies. Second, a selective recognition of the introduced biotin-label by solid-supports, e.g. magnetic beads or affinity matrices that bear, for example, streptavidin as a high-affinity biotin-binding molecule to facilitate an immobilization of the targeted compound. Such methods are known with a great number of variants in the chemistry of biotin and streptavidin as well as in the number and type of solid-supports.

For some applications, the major drawback of the high-affinity interaction between biotin and streptavidin is the almost irreversible nature at physiological conditions. In order to dissociate the two binding partners, extreme denaturing conditions are required, which usually inactivates the biological activity of the biotin-labeled biomolecules. This fact in particular renders the separation of living cells difficult.

To improve the release properties of biotin/streptavidin affinity systems, chemically modified biotin and streptavidin derivatives have been introduced, wherein only one or even both binding partners were modified. Such modifications lower the stability of the biotin/streptavidin complex by several orders of magnitude and thereby facilitate the dissociation of the two binding partners (see for example US 20080255004). Furthermore, mutated streptavidin proteins have been developed with reduced affinity to biotin or its analogues (M. Qureshi et al, J. Biol. Chem. 276 (2001) 46422-46428; T. Sano et al., Proc. Natl. Acad. Sci. USA 92 (1995) 3180-3184). US 5506121 discloses so called "strept-tags" i.e. peptides with reduced binding affinity to streptavidin.

The modification of biotin or streptavidin is laborious. Since many reagents for the coupling of biotin with substrates like antibodies are commercially available, it is widely spread to use unmodified biotin as labeling agent.

In this respect, US 5215927 describes the isolation of target cells by contacting the desired cell population with a monoclonal antibody and subsequent incubation with a biotinylated antispecies immunoglobulin directed to the specific monoclonal antibody. The mixture is separated over a solid-phase comprising immobilized avidin as biotin-binding molecule, which facilitates the immobilization of the target cells and separation of unlabelled targets. The desired cells are subsequently released by mechanical agitation to disrupt the immobilized complex.

The use of release mechanisms mediated by, e.g. unselective enzyme degradation, chemical reactions, intense mechanical forces, high temperature, or strong saline conditions are undesirable for the separation of living cells, because it is important to preserve the cells integrity and viability. Accordingly, mechanisms are desired that allow a rapid and selective release of the target cells at physiological conditions.

In alternative to enzymatic or chemical cleavage or the use of modified biotin/streptavidin molecules, biotin/streptavidin systems can also be cleaved by a ligand competition mechanism. For example, a biotinylated molecule can be released from a streptavidin-support by adding an excess of free biotin in order to replace the biotinylated molecule.

Methods based on the competition of free biotin or streptavidin against the respective counterpart (streptavidin or biotin) are known in numerous variants. James Hirsch et al. give an overview of these techniques in Analytical Biochemistry 308 (2002) 343-357.

The competition reaction of free biotin/streptavidin against the respective bounded counterpart is disclosed in WO 92/16841 for analytical means. WO 92/16841 describes inter alia a method for detecting a reporter molecule, which is specifically bound to an analyte and an insoluble phase via a streptavidin/biotin-binding system. After the work-up procedure, the streptavidin/biotin-binding system is cleaved by a displacement ligand and the released analyte is analytically detectable via a reporter molecule.

US 5518882 discloses a similar method, wherein target cells are bound via a biotin-binding system to an insoluble phase provided by magnetic particles. This allows a further enrichment by applying a magnetic field, which immobilizes the target cells coupled to the magnetic beads. The target cells can be released from the particles by cleaving the biotin-binding system with a displacement ligand. Preferable, the conjugate "(magnetic bead)-biotin-antibody-cell" is cleaved by adding free streptavidin in access, resulting in a "(magnetic bead)-biotin-streptavidin-conjugate" and an "antibody-cell-complex".

In general, a competition reaction, i.e. the displacement of a first ligand with a second ligand will only proceed

until the equilibrium between the kinetics of the binding reaction of the first and second ligand is reached. The equilibrium depends on the respective binding forces and concentrations of the ligand and the thermodynamic conditions of the reaction. The known competition reactions to displace biotin by streptavidin therefore lead either to an uncompleted release or are difficult to control since the underlying kinetics are usually not known.

US 2008/0255004, US 6869606, and US 4656252 disclose biotinylated antibodies comprising modified biotin, wherein the biotin moiety and the antibody moiety of the biotinylated antibodies are separated by a spacer group consisting of an aryl, alkyl, or aminocaprolic acid group. The use of hydrophobic aryl or alkyl groups is expected to cause aggregation in aqueous solutions. Since physiological conditions of biological systems usually require aqueous solutions, agglomeration is an eminent problem for techniques utilizing rather hydrophobic substances. Spacer molecules derived from amino caprolic acid (so called "LC linker") possess a linear alkyl chain with residues that support homo- or heterofunctional bioconjugation chemistries. However, the hydrophobic properties of these alkyl linkers and spacers are most often characterized by increased aggregation and precipitation of the modified or linked products.

**Summary of the invention**

It was therefore an object of the present invention to provide a reliable releasable conjugate comprising a biotinylated ligand, like an antibody, and a releasable molecule binding to the biotin moiety of the biotinylated ligand.

Surprisingly, it was found that the binding affinity of a binding partner targeted to the biotin moiety of a biotinylated ligand can be modified by placing a biocompatible spacer molecule between the biotin moiety and the biotinylated ligand.

Object of the invention was therefore a releasable conjugate comprising a biotinylated ligand ($Ligand_1$) having a biotin moiety, a ligand moiety and a biotin-binding molecule (bbm) bound to the biotin moiety of the biotinylated ligand characterized in that the biotin moiety and the ligand moiety of the biotinylated ligand are separated by a spacer group consisting of polyethylene glycol according to the general formula I

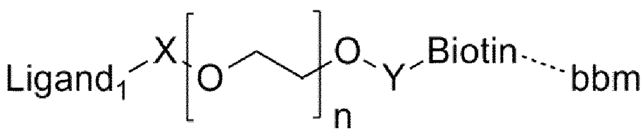

I

with n = 1 - 500, and X, Y = same or different, substituted or unsubstituted alkyl groups with 1 to 20 carbon atoms.

The releasable conjugate is dissociated by the treatment with a release agent, which disrupts the interaction between the biotin and the biotin-binding molecule.

In the releasable conjugate, groups X and Y can stand for substituted alkyl groups having 1 to 20 carbon atoms having at least one or more amine, amide, and/or thioether residue.

The releasable conjugate of the invention is prepared by coupling a biotinylated ligand comprising a spacer group consisting of polyethylene glycol between the biotin and the ligand moiety with a biotin-binding molecule. The biotinylated ligand comprising the spacer group consisting of polyethylene glycol units might be coupled with one or several biotin-binding molecules and *vise versa* resulting in a releasable conjugate having one or more releasable cleavage sites. Accordingly, releasable conjugates of the invention may comprise biotinylated ligands, especially biotinylated antibodies carrying 1 to 20 biotin-binding molecules (bbm).

Preferable, the number n of glycol units in the polyethylene spacer is between 1 and 100, more preferred between 1 and 25, and most preferred between 1 and 10.

In a first, second or third embodiment of the invention, the releasable conjugate has the structure according to general formula IIa, IIb or IIc. In these embodiments, X and/or Y stand for the shown substituted residues having 1 to 20 carbon atoms and n for the number of glycol units as already disclosed.

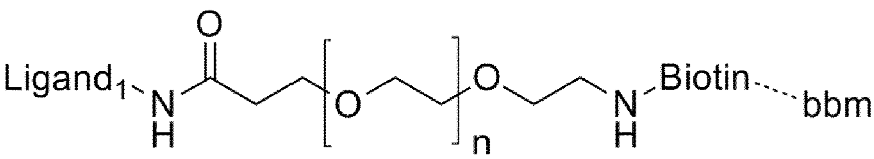

IIa

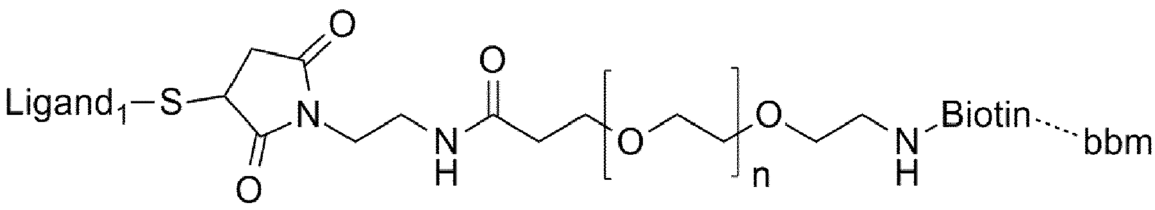

IIb

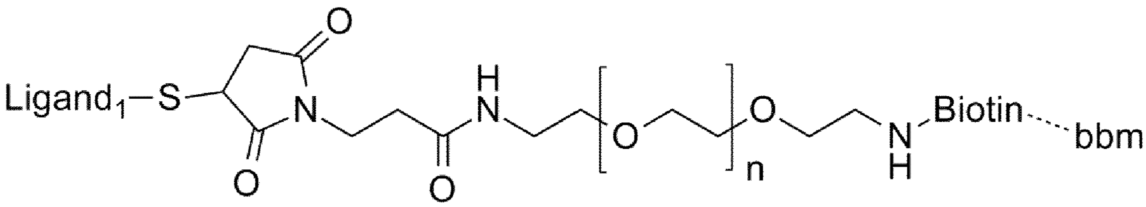

IIc

Fig.1 shows conjugates according to the prior art containing Biotin (1), LC-Biotin (2), LC-LC-Biotin (3) (not according to the invention), and PEO-Biotin (4) (according to the invention). LC stands for "Long chain", a residue based on 6-aminocaproic acid; LC-LC stands for a double 6-aminocaproic acid spacer, while PEO stands for polyethylene oxide also known as polyethylene glycol.

As shown in Fig. 1, the biotin-binding molecule can be an antibody, which is linked to a microbead i.e. a solid-phase particle.

The term "biotin" refers to 5-[(3a*S*,4*S*,6a*R*)-2-oxohexahydro-1*H*-thieno[3,4-*d*]imidazol-4-yl]pentanoic acid and its derivatives supporting host/guest interactions. Such compounds include, for example, iminobiotin, desthiobiotin, and DSB-X biotin, while the preferred molecule in this invention is biotin.

The term "ligand" refers any kind of biomolecule that possesses the capability of binding to other biomolecules. Ligands are especially biomolecules selected from the group consisting of antibodies, proteins, peptides, nucleic acids, carbohydrates, lipids, and derivatives thereof. Preferable, the term "ligand" refers to an antibody directed against cells having an antigen selected from the group consisting of CD3, CD4, CD8, CD14, CD19, CD25, CD34, CD56, and CD133. Accordingly, the term "biotinylated ligand" refers to covalently linked conjugates of biotin and any kind of biomolecule that possesses the capability of binding to other biomolecules.

The term "biotin-binding molecule" (bbm) refers to a biomolecule displaceable from the biotin moiety of the biotinylated ligand by biotin, streptavidin or derivates thereof. Preferable, the biotin-binding molecule (bbm) is an antibody, especially directed against cells having an antigen selected from the group consisting of CD3, CD4, CD8, CD14, CD19, CD25, CD34, CD56, and CD133. Furthermore, the biotin-binding molecule used in this invention can be an antibody selected from the class of immunoglobulin, e.g. IgG, IgA, IgM, IgD, IgE derived from animals, like e.g. mice, monkeys, goats, rabbits, sheep, or lamas. The term "biotin-binding molecule" (bbm) also relates to fully intact or fragmented antibody derivatives, e.g., Fab, Fab', F(ab')2, sdAb, scFv, di-scFv that have been synthesized by recombinant procedures including covalent and non-covalent conjugates containing these kind of molecules. In the releasable conjugate according to the invention a release agent can dissociate the biotin-binding molecule from the biotin moiety.

The term "release agent" refers to any compound capable of binding to the biotin moiety of the biotinylated ligand or the biotin-binding molecule thereby disrupting the interaction between the biotin moiety and the biotin-binding molecule. Suitable release agents are compounds, e.g., streptavidin, avidin, biotin, and/or derivatives thereof. The preferred release agent in this invention is biotin. Suitable-modified streptavidin molecules are disclosed by James Hirsch et al. in Analytical Biochemistry 308 (2002) 343-357. Accordingly, the term "streptavidin and derivates thereof" refers to any molecule derived from streptavidin providing a binding affinity to biotin that is comparable to the binding affinity of unmodified streptavidin to biotin as well as to derivatives and conjugates of streptavidin without having the purpose of especially lowering the binding affinity to biotin.

The releasable conjugate according to general formula I may contain a biotinylated antibody as ligand, X, Y as already disclosed, and n = 1 - 500 as shown in general formula III.

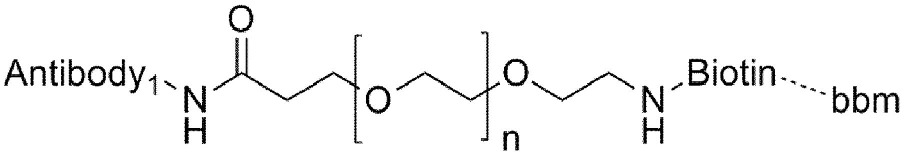

III

In a first variant of the invention, the ligand, the biotin-binding molecule (bbm) that binds to the biotin moiety and/or the release agent possess a label that can emit a detection signal, e.g., comprises at least one fluorescent, chemiluminescent, or radioactive unit. Suitable labeled release agents are, for example, commercial available under the trade names "Anti-Biotin-PE" and "APC Streptavidin" at Miltenyi Biotec GmbH, Germany and BioLegend Inc., respectively.

The use of labeled release agents or labeled ligands enables a quantitative detection of respective compounds and/or of targeted cells.

In a second variant of the invention, the biotin-binding molecule (bbm) is immobilized i.e. attached to a solid-support. The solid-support may be any of the known systems in biotechnology for immobilizing cells and can have the shape of particles, for example, sheets, plates, membranes, tubes, columns, wells, or micro arrays manufactured from various materials like polystyrene (PS), polymethylmethacrylate (PMMA), polyvinyl toluene (PVT), polyethylene (PE), or polypropylene (PP). Suitable materials are commercially available.

The solid support can further be a nano- to microscale magnetic particle, known in the art as magnetic beads. The mean diameter of the beads can range from 10 nm to 10 μm. Biocompatible magnetic particles are commercially available and consist of, for example, forms of magnetically iron oxide coated by a shell of dextran molecules or silica. The solid support may also be polymers containing magnetic materials. Suitable particles are commercial available from Miltenyi Biotec GmbH, Germany under the trade name "MicroBeads" and "MACSiBEADS" possessing a hydrodynamic diameter of 50-100 nm and 3-4 μm, respectively.

According to this invention, magnetic particles are used as solid-support to facilitate magnetic separation processes. For example, the biotin-binding molecule (bbm) can be coupled to a magnetic particle via a covalent coupling procedure. In this respect, the magnetic particles would act as a solid-support in separation protocols. Suitable magnetic particles conjugated, for example, with anti-biotin antibodies and respective apparatus that support magnetic field separations of living cells are available from Miltenyi Biotec GmbH, Germany.

In a third variant of the invention, the biotin-binding molecule (bbm) might be an IgG antibody, for example, an anti-biotin antibody. This IgG antibody may than be recognized by a second antibody, for example, by an anti-IgG antibody, which can be conjugated to a solid-support as already disclosed. Supported anti-IgG antibodies are commercial available from Miltenyi Biotec GmbH as "Anti-IgG MicroBeads". Such a releasable conjugate is illustrated in general formula IV, with antibody$_1$ targeted to a biomolecule of interest, antibody$_2$ as biotin-binding molecule, and antibody$_3$ binding to antibody$_2$.

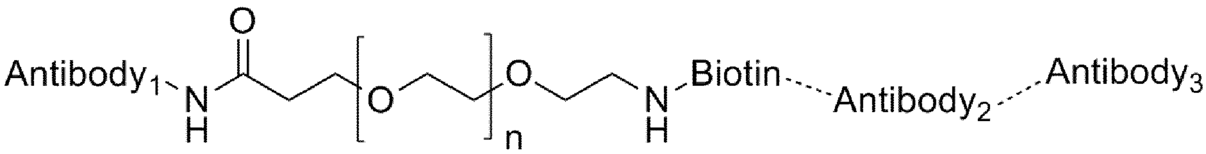

IV

Another object of the invention is a method for cleaving a releasable conjugate comprising a biotinylated ligand (Ligand$_1$) having a biotin moiety, a ligand moiety and a biotin-binding molecule (bbm) bound to the biotin moiety of the biotinylated ligand, wherein the biotin moiety and the ligand moiety of the biotinylated ligand are separated by a spacer group consisting of polyethylene glycol according to the general formula I

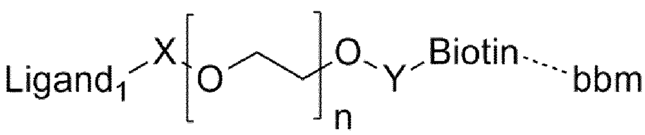

I

with n = 1 - 500, and X, Y = X, Y = same or different, substituted or unsubstituted alkyl groups with 1 to 20 carbon atoms by providing biotin, streptavidin or derivates thereof in a sufficient concentration to displace the biotin-binding molecule (bbm) from the biotin moiety of the biotinylated ligand.

All embodiments and variants of the already disclosed releasable conjugate can be employed in the method of the invention.

The method of the invention results in the dissociation of the releasable conjugate between the biotin moiety and the biotin-binding molecule (bbm) as shown in figures V and VI. For example, if an anti-biotin antibody is used as biotin-binding molecule (bbm), the releasable conjugate is selectively cleaved between the biotin moiety and the anti-biotin antibody by adding an excess of free biotin as release agent. The release agent forms a non-covalent complex with the biotin-binding molecule (bbm) and facilitates the release of the biotinylated ligand (V).

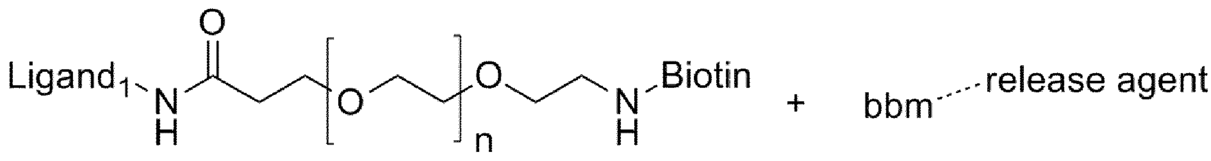

V

In alternative, the utilization of an excess of, e.g., Streptavidin as release agent forms a non-covalent complex with the biotinylated ligand, which results in a displacement of the biotin-binding molecule (VI).

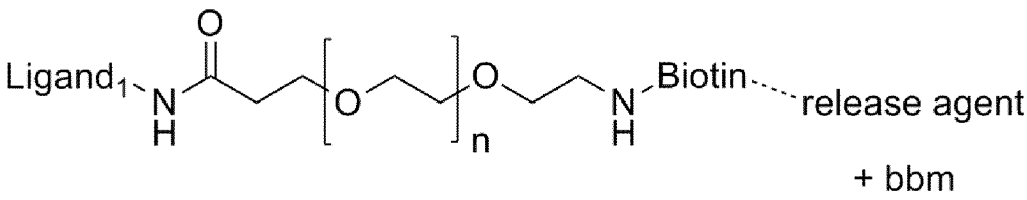

VI

The method of the invention is based on a competition reaction and will proceed until the binding equilibrium between the release agent and the biotin-binding molecule (bbm) or accordingly to the biotin moiety is reached. The equilibrium depends on the respective binding forces, the concentration of the free release agent, and the thermodynamic conditions of the reaction. Accordingly, the term "sufficient amount to displace the second ligand by streptavidin or derivates thereof" does not stand for a specific value but needs to be evaluated according to the desired release rate.

For example, by utilizing an anti-biotin antibody as biotin-binding molecule (bbm) the releasable conjugate of the invention can be selectively cleaved by utilizing free biotin or streptavidin as release agent. If free biotin is used as release agent, a molar ratio (biotinylated ligand : free biotin) of 1:1,000 to 1:1,000,000, preferable a molar ratio of 1:1,000 to 1:100,000 is sufficient to dissociate the releasable conjugate. In case of streptavidin as release agent, a molar ratio (biotinylated ligand : streptavidin) of 1:1 to 1:10,000 preferable 1:10 to 1:1,000 may be provided to dissociate the releasable conjugate.

In another embodiment of the method of the invention, it was found that the addition of an auxiliary release agent improves the release efficiency method of the invention. In this embodiment, streptavidin or derivates thereof and an auxiliary release agent having the general formulas VII, VIII, or IX

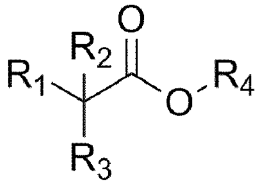

VII

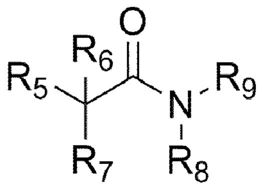

VIII

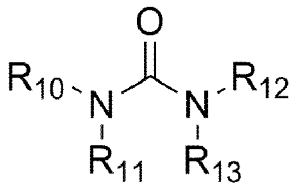

IX

with $R_{1-3,5-13}$ = same or different hydrogen, substituted, or unsubstituted alkyl residues having 1-20 carbon atoms and $R_4$ = substituted or unsubstituted alkyl residues having 1-20 carbon atoms are provided in a sufficient concentration to displace the biotin-binding molecule (bbm) from the biotin moiety of the biotinylated ligand.

The following compounds 7-15 illustrate specific examples of auxiliary release agents according to the invention. Not suitable as auxiliary release agent according to the invention is, for example, compound 15 despite its similar molecular structure in comparison to compounds 7 -14.

| No. | chemical structure |
|---|---|
| 7 | 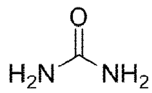 |
| 8 | 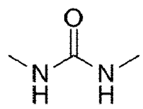 |
| 9 | 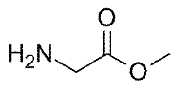 |
| 10 | 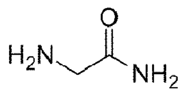 |
| 11 | 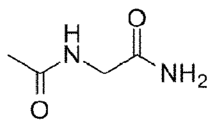 |
| 12 | 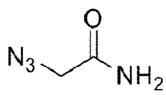 |
| 13 | 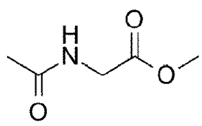 |
| 14 | 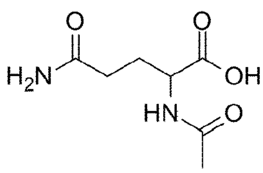 |
| 15 | 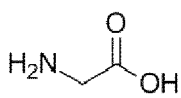 |

In this variant of the invention, the person skilled in the art can easily evaluate the suitable concentration of the auxiliary release agent with respect to the desired release rate. The examples provide some insights regarding suitable conditions and resulting release efficiencies. In praxis, the auxiliary release agent can be used in an high excess in comparison to the release agent, for example with a molar ratio of 1:1,000 to 1:1,000,000 (release agent : auxiliary release agent).

It is furthermore an obj ect of the invention to provide a kit comprising a biotinylated antibody, a biotin binding molecule, a release agent and optionally an auxiliary release agent. The biotinylated antibody is selected according to the desired target cells, e.g., against a certain antigen. The kit according to the invention is used by first labeling the desired cells with the biotinylated antibody, then conjugating the biotin-binding molecule, which is optionally labeled or coupled to a solid-support. After performing an isolation/purification step to yield the desired target cells, the release agent and optionally the auxiliary release agent is added to cleave the biotinylated antibody with the target cells from

the biotin-binding molecule.

**Examples**

The examples and comparative examples show that the releasable conjugates and the method according to the invention allow a fast and reliable dissociation. Cells targeted by the releasable conjugates can be separated from cell suspensions and efficiently released, which enables further processing of the isolated cells.

Example 1 - Coupling of anti-CD8-Biotin conjugates and cell surface staining:

Coupling protocol 1:

To prepare biotinylated conjugates according to the first embodiment, IIa anti-CD8-antibody in PBS/EDTA-buffer was re-buffered to 100 mM $NaHCO_3$-buffer (pH 8.3). NHS-Biotin, NHS-LC-Biotin, NHS-LC-LC-Biotin, and NHS-PEO-Biotin (n=4, as shown in Fig.1, available from Thermo Scientific/Pierce) were dissolved in DMSO at concentration of 5 mg/mL and added in different molar ratios to the antibody solution at 2.5 mg/mL. After 1 h incubation time at room temperature, unreacted biotin was removed by gel filtration utilizing PBS/EDTA-buffer. Protein concentrations were determined by the absorbance at 280 nm using experimentally determined extinction coefficients. The approximate biotin to protein ratios (B/P) were determined by the standardized HABA-avidin-assay.

Coupling protocol 2:

To prepare biotinylated conjugates according to the second embodiment IIb or IIc anti-CD8-antibody was reduced with 10 mM DTT in MES-buffer. After 1 h incubation time at room temperature, the antibody was purified by gel filtration utilizing PBS/EDTA-buffer. To afford the anti-CD8-PEO-Biotin (5) (according to IIb) and anti-CD8-$PEPO_2$-Biotin (6) (according to IIc) conjugates, maleimide-PEO-Biotin or maleimide-$PEO_2$-Biotin (available from Thermo Scientific/Pierce), respectively, were dissolved in PBS/EDTA-buffer at 5 mg/mL and added with a molar excess to the antibody solution at 2.5 mg/mL. After 15 h incubation time at room temperature, the unreacted biotin was removed by gel filtration utilizing PBS/EDTA-buffer. Protein concentrations were determined by the absorbance at 280 nm using experimentally determined extinction coefficients. The approximate biotin to protein ratios (B/P) were determined by the standardized HABA-avidin-assay.

Cell surface staining with anti-CD8-Biotin conjugates: peripheral blood mononuclear cells (PBMCs) in PBS/EDTA/BSA-buffer were stained for 10 min at 4 °C with 0.5 μg/mL of anti-CD8-Biotin (1), anti-CD8-LC-Biotin (2), anti-CD8-LC-LC-Biotin (3), or anti-CD8-PEO-Biotin (4) as illustrated in Fig. 1. The cells were washed with cold PBS/EDTA/BSA-buffer and stained for 10 min at 4 °C with an excess of anti-Biotin-PE. The cells were washed with cold PBS/EDTA-BSA-buffer and analyzed by flow cytometry.

Fig. 2 shows the mean fluorescence intensities that have been achieved with different anti-CD8-Biotin conjugates as a function of the B/P ratio. Cell surface staining with anti-CD8-LC-Biotin (2), anti-CD8-LC-LC-Biotin (3), or anti-CD8-PEO-Biotin (4) furnished equally fluorescence signals. In contrast, cells stained with anti-CD8-Biotin (1) missing a spacer molecule between the biotin and the antibody moiety provided diminished mean fluorescence intensity although the B/P ratio of the biotinylated conjugates was comparable among each other. These experiments illustrate the advantage of affinity systems containing spacer molecules as in anti-CD8-LC-Biotin (2), anti-CD8-LC-LC-Biotin (3), or anti-CD8-PEO-Biotin (4) in comparison to conjugates, like anti-CD8-Biotin (1) missing an additional linker between the biotin and antibody moiety.

Example 2 - Cell surface staining with anti-CD8-Biotin conjugates and kinetic analysis:

PBMCs in PBS/EDTA/BSA-buffer were stained for 10 min at 4 °C with 0.5 μg/mL of anti-CD8-Biotin (1), anti-CD8-LC-Biotin (2), anti-CD8-LC-LC-Biotin (3), anti-CD8-PEO-Biotin (4), anti-CD8-PEO-Biotin (5), or anti-CD8-$PEO_2$-Biotin (6). The cells were washed with cold PBS/EDTA/BSA-buffer and stained for 10 min at 4 °C with an excess of anti-Biotin-PE. The cells were washed with cold PBS/EDTA-BSA-buffer and incubated with 0.1 mM biotin at room temperature in the dark. The cells and the dissociation of anti-Biotin-PE was monitored by flow cytometry analysis after certain time points in relation to the starting sample missing release agent.

Fig. 3 illustrate a fast decrease of the fluorescence signal of CD8 positive cells within the first 10 min reaction time in the presence of anti-CD8-Biotin (1), anti-CD8-PEO-Biotin (4), anti-CD8-PEO-Biotin (5), or anti-CD8-$PEO_2$-Biotin (6), while the dissociation rate in the case of anti-CD8-LC-Biotin (2) and anti-CD8-LC-LC-Biotin (3) was significant lower. These examples show that the release of conjugates comprising either no spacer molecule or spacer units according to the invention is much faster than with conjugates comprising a spacer molecule not according to the invention, like

LC or LC-LC. It is important to note, that spacer molecules seem to be necessary to achieve equally cell stainings as already disclosed in Fig. 2.

Example 3 - Cell surface staining with anti-CD8-PEO-Biotin conjugate and kinetic analysis:

CD8 positive cells in PBS/EDTA/BSA-buffer were stained for 10 min at 4 °C with 0.5 μg/mL anti-CD8-PEO-Biotin (4). The cells were washed with cold PBS/EDTA/BSA-buffer and stained 10 min at 4 °C with an excess of anti-Biotin-PE. The cells were washed with cold PBS/EDTA-BSA-buffer and incubated with different concentrations of the compounds 10, 12, and 15 at room temperature in the dark. The dissociation of the CD8-PEO-Biotin/anti-Biotin-PE system was monitored by flow cytometry analysis after 10 min incubation time in relation to samples missing release agent.

Fig. 4 illustrates that the compounds 10 and 12 highly facilitate the dissociation of the CD8-PEO-Biotin/anti-Biotin-PE system in comparison to molecule 15, furnishing lower release yields. These examples show that auxiliary release agents according to the invention can facilitate the dissociation of the invented releasable conjugates.

Example 4 - Cell surface staining with anti-CD8-Biotin conjugates and kinetic analysis:

CD8 positive cells in PBS/EDTA/BSA-buffer were stained for 10 min at 4 °C with 0.5 μg/mL anti-CD8-LC-LC-Biotin (3) and anti-CD8-PEO-Biotin (4). The cells were washed with cold PBS/EDTA/BSA-buffer and stained for 10 min at 4 °C with an excess of anti-Biotin-PE. The cells were washed with cold PBS/EDTA-BSA-buffer and incubated with different amounts of streptavidin in absence or in combination with compound 12 at 100 mM concentration. The dissociation of the CD8-PEO-Biotin/anti-Biotin-PE and the CD8-LC-LC-Biotin/anti-Biotin-PE systems were monitored by flow cytometry analysis after 10 min incubation times in relation to samples missing release agent.

Fig. 5 shows that the CD8-PEO-Biotin/anti-Biotin-PE system dissociates significantly faster than the CD8-LC-LC-Biotin/anti-Biotin-PE system in the presence of streptavidin. Moreover, the addition of compound 12 as auxiliary release agent accelerates the streptavidin-mediated dissociation process. This example shows that streptavidin on its own and in combination with compound 12 as auxiliary release agent can facilitate the dissociation of conjugates containing a spacer molecule according to the invention. In contrast, the dissociation of affinity systems possessing a spacer molecule not according to the invention, like LC-LC, is significantly less influenced by the utilization of the release agents.

Example 5 - Cell surface staining with anti-CD8-Biotin conjugates and kinetic analysis:

CD8 positive cells in PBS/EDTA/BSA-buffer were stained for 10 min at 4 °C with 0.5 μg/mL of anti-CD8-LC-LC-Biotin (3) and anti-CD8-PEO-Biotin (4). The cells were washed with cold PBS/EDTA/BSA-buffer and stained for 10 min at 4 °C with an excess of anti-Biotin-PE. The cells were washed with cold PBS/EDTA-BSA-buffer and incubated with different concentrations of allophycocyanin (APC)-labeled streptavidin (Biolegend, Inc.) in absence or in combination with compound 12. The dissociation of the CD8-PEO-Biotin/anti-Biotin-PE and the CD8-LC-LC-Biotin/anti-Biotin-PE systems were monitored by flow cytometry analysis after 10 min incubation times in relation to samples missing release agent.

Fig. 6 shows that the CD8-PEO-Biotin/anti-Biotin-PE system dissociates significantly faster that the CD8-LC-LC-Biotin/anti-Biotin-PE system in the presence of APC-streptavidin. Moreover, the addition of compound 12 as auxiliary release agent accelerates the APC-streptavidin-mediated dissociation process. This example shows that APC-streptavidin on its own and in combination with compound 12 as auxiliary release agent can facilitate the dissociation of releasable conjugates containing a spacer molecule according to the invention. In contrast, the release agents significantly less influence the dissociation of affinity systems possessing a spacer molecule not according to the invention, like the CD8-LC-LC-Biotin/anti-Biotin-PE system.

Example 6 - Magnetic cell separation with anti-CD8-PEO-Biotin conjugate:

PBMCs in PBS/EDTA/BSA-buffer were incubated at 4 °C for 10 min with anti-CD8-LC-LC-Biotin (3) and anti-CD8-PEO-Biotin (4). The cells were washed with cold PBS/EDTA/BSA-buffer and incubated for 15 min at 4 °C with anti-Biotin-MicroBeads (Miltenyi Biotec GmbH) and for 10 min at 4 °C with anti-CD8-PE. The cells were washed with cold PBS/EDTA-BSA-buffer and resuspended in 500 μL of cold buffer. The cell suspension was applied on a MS-column (Miltenyi Biotec GmbH) in a magnetic field for magnetic cell separation. The flow-through was collected as magnetically unlabeled cell fraction. The cells were washed within the magnetic field and the column was removed from the separator prior to the elution of the cells with 1 mL of cold PBS/EDTA/BSA-buffer. The isolated cell fraction was incubated for 10 min at different concentrations of biotin. The cell suspension was applied onto a second column and flow-through was collected as eluted cells. Cells retained on the column were eluted with 500 μL of cold PBS/EDTA/BSA-buffer in absence of the magnetic field. The amount of CD8 positive cells in the isolated fractions was determined by flow cytometry

analysis. The percentage allocation of the eluted cells was calculated as follows:

$$\frac{\text{amount of cells in the eluted fraction}}{\text{amount of cells in the eluted fraction} + \text{amount of cells in the fraction retained by magnetic field}}$$

Fig. 7 illustrates that the incubation of cells labeled by the CD8-PEO-Biotin/anti-Biotin-MicroBead system according to the invention with biotin leads to a significantly better release of the magnetic label in comparison to the CD8-LC-LC-Biotin/anti-Biotin-MicroBead system. This example shows the possibility to efficiently release the magnetic labeling of separated cells in the CD8-PEO-Biotin/anti-Biotin-MicroBead system by the addition of biotin as release agent.

<u>Example 7 - Magnetic cell separation with anti-CD8-PEO-Biotin conjugate:</u>

PBMCs in PBS/EDTA/BSA-buffer were incubated for 10 min at 4 °C with anti-CD8-LC-LC-Biotin (3) and anti-CD8-PEO-Biotin (4). The cells were washed with cold PBS/EDTA/BSA-buffer and incubated for 15 min at 4 °C with anti-Biotin-MicroBeads (Miltenyi Biotec GmbH) and for 10 min at 4°C with anti-CD8-PE. The cells were washed with cold PBS/EDTA-BSA-buffer and resuspended in 500 μL of cold buffer. The cell suspension was applied on a MS-column in a magnetic field for magnetic cell separation. The flow-through was collected as magnetically unlabeled cell fraction. The cells were washed within the magnetic field and the column was removed from the separator prior to elution of the cells with 1 mL of cold PBS/EDTA/BSA-buffer. The isolated cell fraction was incubated for 10 min at different concentrations of streptavidin with and without compound 12. The cell suspension was applied onto a second column and flow-through was collected as eluted cells. Cells retained by the column were eluted with 500 μL of cold PBS/EDTA/BSA-buffer in absence of the magnetic field. The amount of CD8 positive cells in the isolated fractions was determined by flow cytometry analysis. The percentage allocation of the eluted cells was calculated as follows:

$$\frac{\text{amount of cells in the eluted fraction}}{\text{amount of cells in the eluted fraction} + \text{amount of cells in the fraction retained by magnetic field}}$$

Fig. 8 illustrates that the incubation of cells labeled by the CD8-PEO-Biotin/anti-Biotin-MicroBead system according to the invention with streptavidin provides a significantly faster release of the magnetic label in comparison to the CD8-LC-LC-Biotin/anti-Biotin-MicroBead system. Moreover, the addition of compound 12 to streptavidin as release agent further improves the dissociation of the CD8-PEO-Biotin/anti-Biotin-MicroBead system. This example shows that the use of an auxiliary release agent according to the invention can significantly improve the release efficiency.

<u>Example 8 - Magnetic cell separation with anti-CD8-PEO-Biotin conjugate:</u>

PBMCs in PBS/EDTA/BSA-buffer were incubated for 10 min at 4 °C with anti-CD8-LC-LC-Biotin (3) and anti-CD8-PEO-Biotin (4). The cells were washed with cold PBS/EDTA/BSA-buffer and incubated for 15 min at 4 °C with anti-Biotin-MicroBeads and for 10 min at 4 °C with anti-CD8-PE. The cells were washed with cold PBS/EDTA-BSA-buffer and resuspended in 500 μL of cold buffer. The cell suspension was applied on a MS-column in a magnetic field for magnetic cell separation. The flow-through was collected as magnetically unlabeled cell fraction. The cells were washed within the magnetic field and the column was removed from the separator prior to elution of the cells with 1 mL of cold PBS/EDTA/BSA-buffer. The isolated cell fraction was incubated for 10 min at different concentrations of APC-streptavidin with and without compound 12. The cell suspension was applied onto a second column and flow-through was collected as eluted cells. Cells retained by the column were eluted with 500 μL of cold PBS/EDTA/BSA-buffer in absence of the magnetic field. The amount of CD8 positive cells in the isolated fractions was determined by flow cytometry analysis. The percentage allocation of the eluted cells was calculated as follows:

$$\frac{\text{amount of cells in the eluted fraction}}{\text{amount of cells in the eluted fraction} + \text{amount of cells in the fraction retained by magnetic field}}$$

Fig. 9 illustrates that cells labeled by the CD8-PEO-Biotin/anti-Biotin-MicroBead system according to the invention and treated with APC-streptavidin as release agent are significantly faster eluted from the magnetic field in

comparison to cells labeled with the CD8-LC-LC-Biotin/anti-Biotin-MicroBead system. Moreover, the addition of compound 12 to APC-streptavidin as release agent further improves the dissociation of the CD8-PEO-Biotin/anti-Biotin-MicroBead system. These results demonstrate that the use of auxiliary release agents according to the invention can significantly improve the release efficiency.

## Claims

1. Releasable conjugate comprising a biotinylated ligand ($Ligand_1$) having a biotin moiety, a ligand moiety and a biotin-binding molecule (bbm) bound to the biotin moiety of the biotinylated ligand **characterized in that** the biotin moiety and the ligand moiety of the biotinylated ligand are separated by a spacer group consisting of polyethylene glycol according to the general formula I

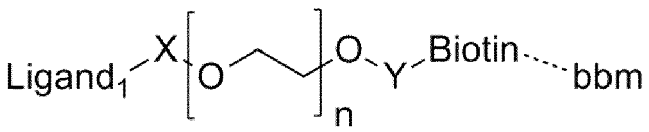

I

with n = 1 - 500, and X, Y = same or different, substituted or unsubstituted alkyl groups with 1 to 20 carbon atoms.

2. Releasable conjugate according to claim 1, **characterized in that** the ligand is a biomolecule selected from the group consisting of antibodies, proteins, peptides, nucleic acids, carbohydrates, lipids, and derivatives thereof.

3. Releasable conjugate according to claim 1 or 2 **characterized in that** the ligand is an antibody directed against cells having an antigen selected from the group consisting of CD3, CD4, CD8, CD14, CD19, CD25, CD34, CD56, and CD133.

4. Releasable conjugate according to any of the claims 1 to 3, **characterized in that** X and Y stand for substituted alkyl groups having 1 to 20 carbon atoms having amine, amide, and/or thioether residues.

5. Releasable conjugate according to any of the claims 1 to 4, **characterized in that** the releasable conjugate has the general formula IIa, IIb, or IIc

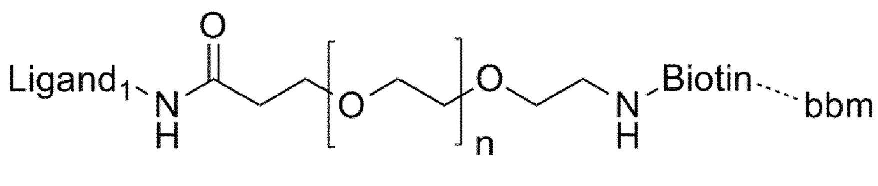

IIa

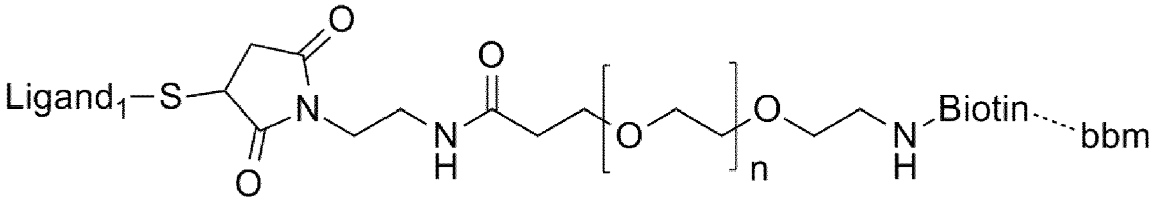

IIb

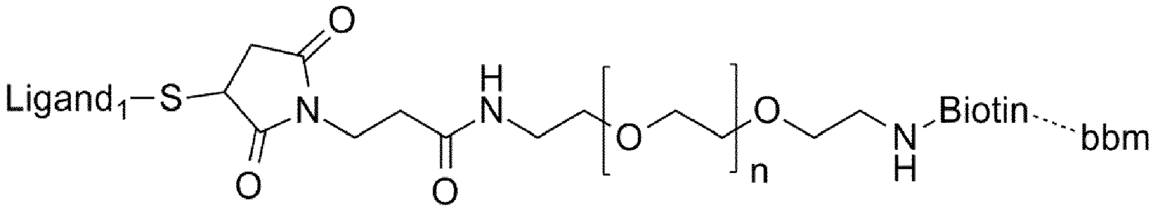

IIc

6. Releasable conjugate according to any of the claims 1 to 5, **characterized in that** the biotin-binding molecule (bbm) is a biomolecule displaceable from the biotin moiety of the biotinylated ligand by biotin, streptavidin or derivates thereof.

7. Releasable conjugate according to any of the claims 1 to 6, **characterized in that** the biotin-binding molecule (bbm) is an antibody.

8. Releasable conjugate according to any of the claims 1 to 7 **characterized in that** the biotin-binding molecule (bbm) is attached to a solid-support.

9. Method for cleaving a releasable conjugate comprising a biotinylated ligand ($Ligand_1$) having a biotin moiety, a ligand moiety, and a biotin-binding molecule (bbm) bound to the biotin moiety of the biotinylated ligand, wherein the biotin moiety and the ligand moiety of the biotinylated ligand are separated by a spacer group consisting of polyethylene glycol according to the general formula I

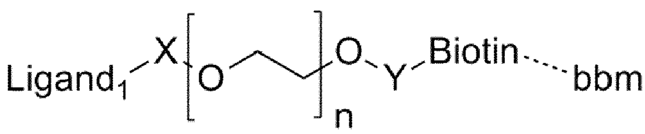

I

with n = 1 - 500, and X, Y = same or different, substituted or unsubstituted alkyl groups with 1 to 20 carbon atoms **characterized in** providing biotin, streptavidin or derivates thereof in a sufficient concentration to displace the biotin-binding molecule (bbm) from the biotin moiety of the biotinylated ligand.

10. Method according to claim 9, **characterized in that** the ligand is a biomolecule selected from the group consisting of antibodies, proteins, peptides, nucleic acids, carbohydrates, lipids, and derivatives thereof

11. Method according to claim 9 **characterized in that** the ligand is an antibody directed against cells having an antigen selected from the group consisting of CD3, CD4, CD8, CD14, CD19, CD25, CD34, CD56, and CD133.

12. Method according to any of the claims 8 to 11, **characterized in that** the biotin-binding molecule (bbm) is a biomolecule displaceable from the biotin moiety of the biotinylated ligand by biotin, streptavidin or derivates thereof.

13. Method according to claim 12, **characterized in that** the biotin-binding molecule (bbm) is an antibody.

14. Method according to any of the claims 8 to 13 **characterized in that** the biotin-binding molecule (bbm) is attached to a solid-support.

15. Method according to any of the claims 8 to 14 **characterized in** providing biotin, streptavidin or derivates thereof and an auxiliary release agent having the general formulas VII, VIII, or IX

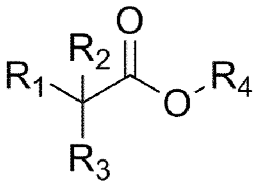 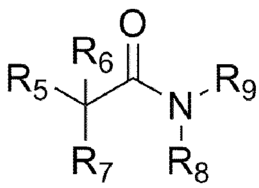 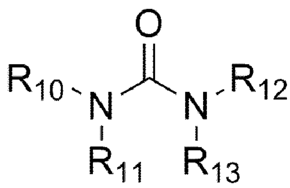

VII VIII IX

with $R_{1-3,5-13}$ = same or different hydrogen, or substituted or unsubstituted alkyl residues having 1 - 20 carbon atoms and $R_4$ = substituted or unsubstituted alkyl residues having 1-20 carbon atoms in a sufficient concentration to displace the biotin-binding molecule (bbm) from the biotin moiety of the biotinylated ligand.

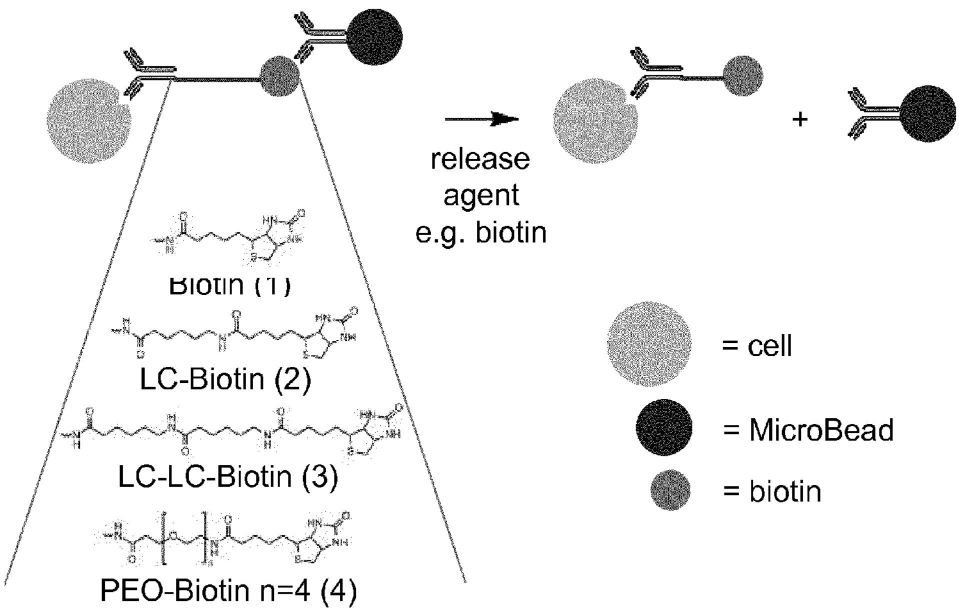
release
agent
e.g. biotin
+
Biotin (1)
LC-Biotin (2)
LC-LC-Biotin (3)
PEO-Biotin n=4 (4)
= cell
= MicroBead
= biotin

Fig. 1

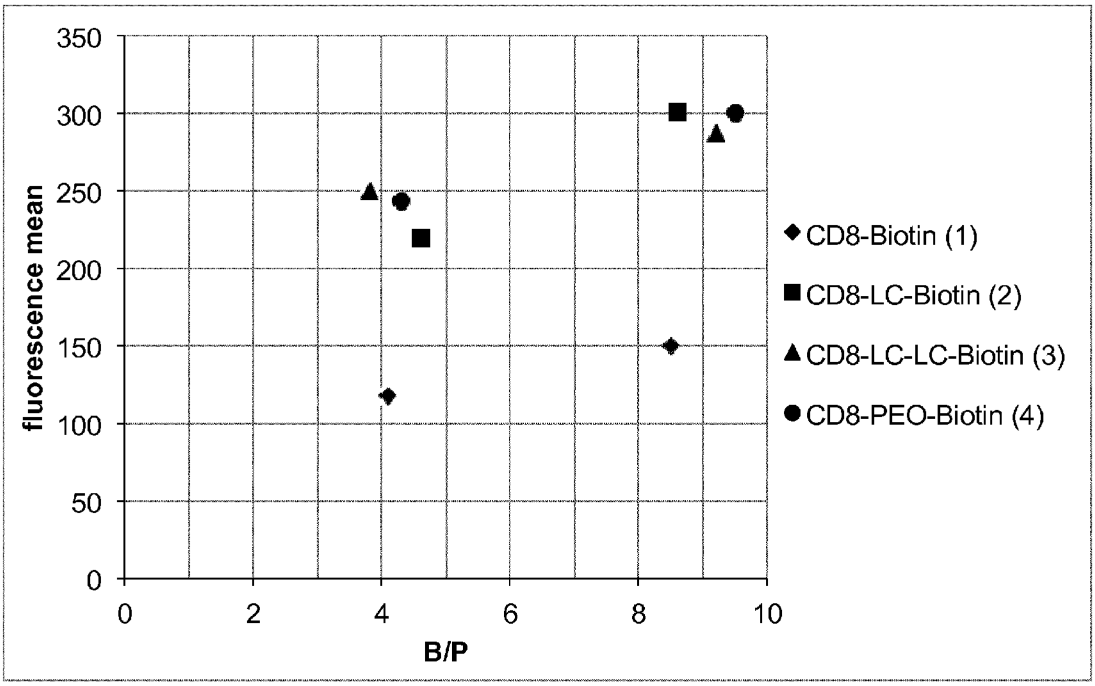
350
300
250
200
150
100
50
0
fluorescence mean
0
2
4
6
8
10
B/P
CD8-Biotin (1)
CD8-LC-Biotin (2)
CD8-LC-LC-Biotin (3)
CD8-PEO-Biotin (4)

Fig. 2:

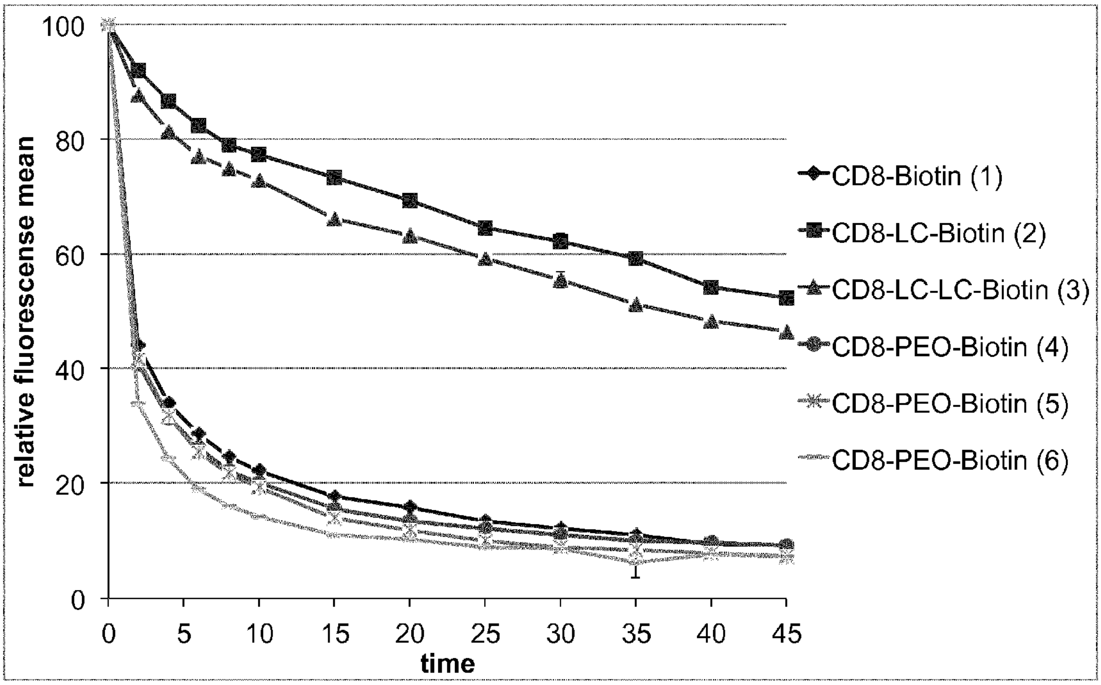
100
80
60
40
20
0
relative fluorescense mean
0
5
10
15
20
25
30
35
40
45
time
CD8-Biotin (1)
CD8-LC-Biotin (2)
CD8-LC-LC-Biotin (3)
CD8-PEO-Biotin (4)
CD8-PEO-Biotin (5)
CD8-PEO-Biotin (6)

Fig. 3:

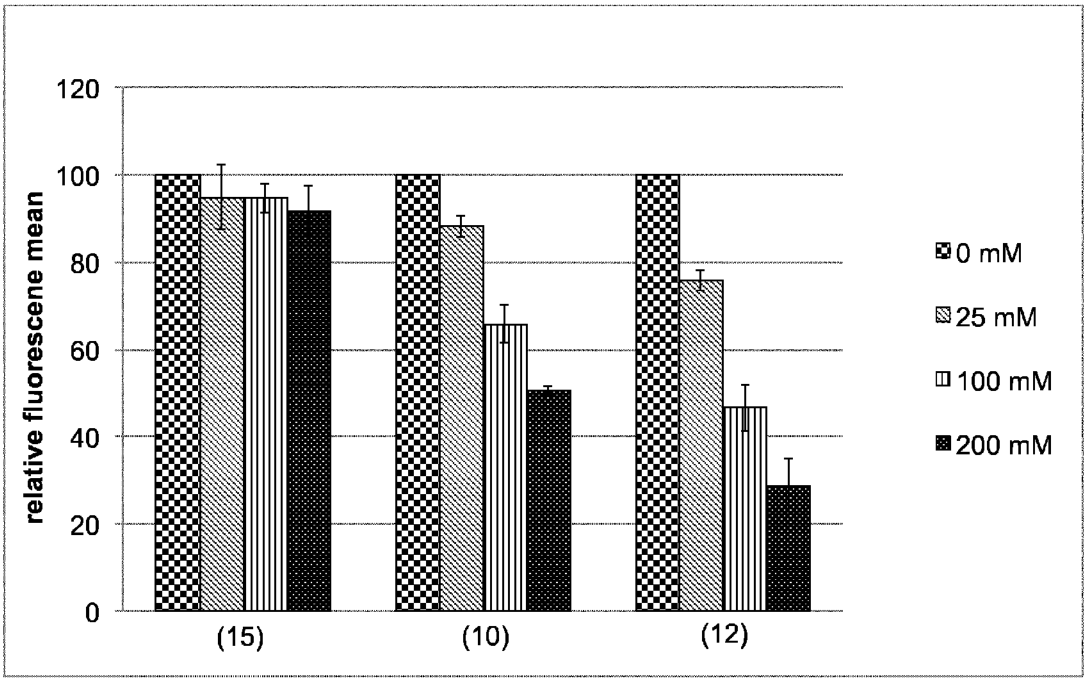
120
100
80
60
40
20
0
relative fluorescene mean
(15)
(10)
(12)
0 mM
25 mM
100 mM
200 mM

Fig. 4:

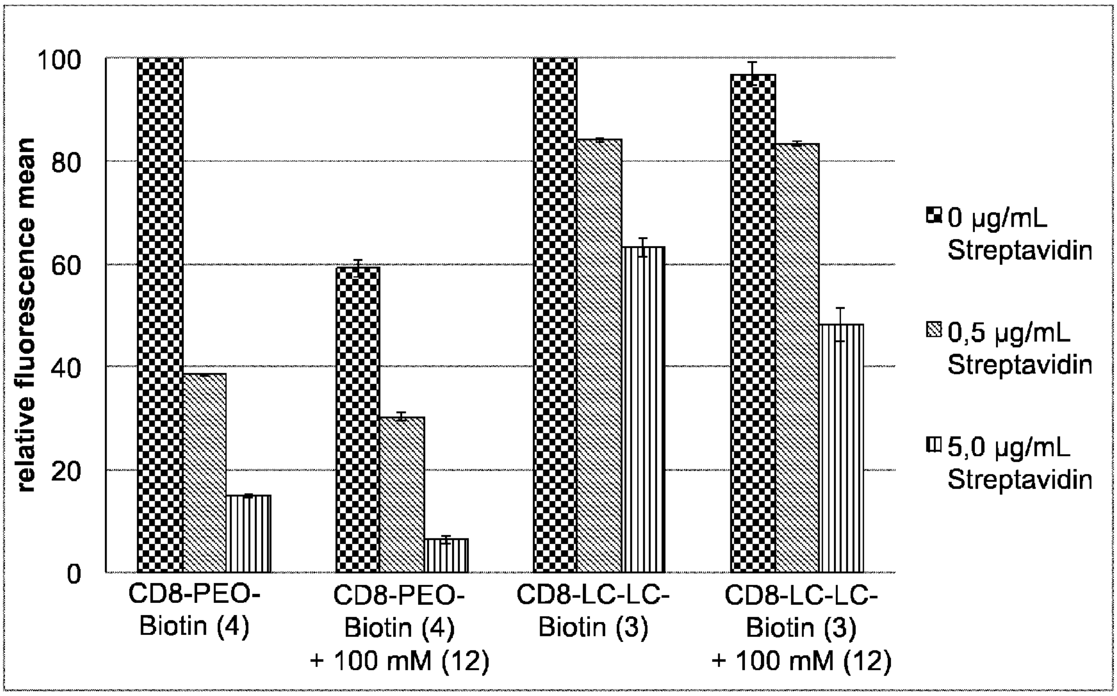
100
80
60
40
20
0
relative fluorescence mean
CD8-PEO-Biotin (4)
CD8-PEO-Biotin (4) + 100 mM (12)
CD8-LC-LC-Biotin (3)
CD8-LC-LC-Biotin (3) + 100 mM (12)
0 µg/mL Streptavidin
0,5 µg/mL Streptavidin
5,0 µg/mL Streptavidin

Fig. 5:

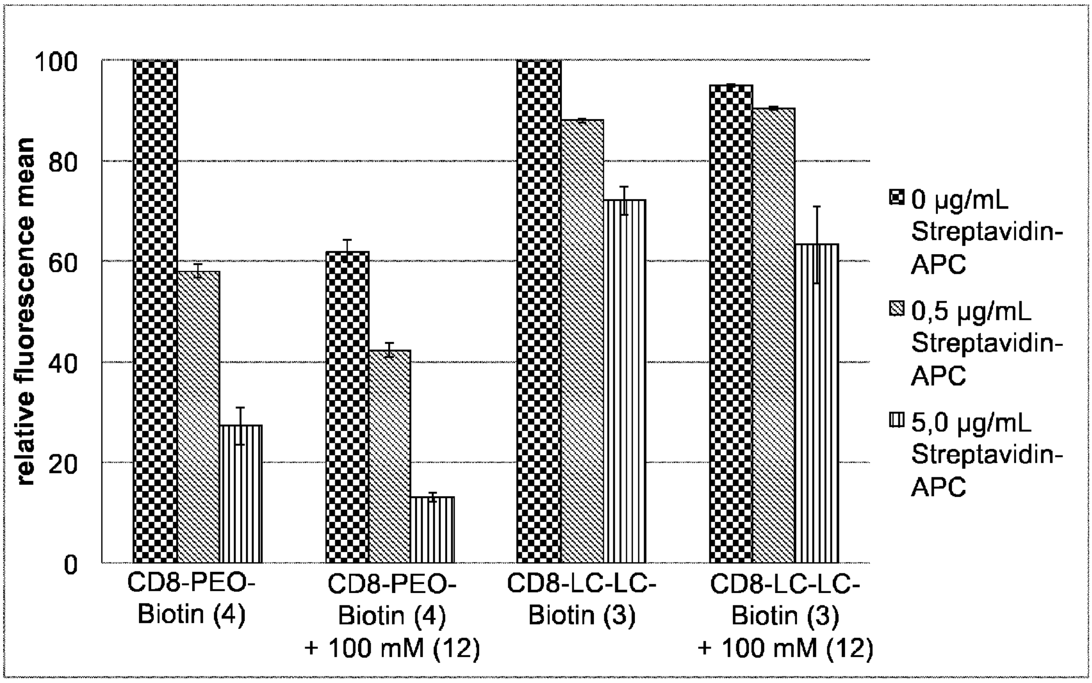
100
80
60
40
20
0
relative fluorescence mean
CD8-PEO-Biotin (4)
CD8-PEO-Biotin (4) + 100 mM (12)
CD8-LC-LC-Biotin (3)
CD8-LC-LC-Biotin (3) + 100 mM (12)
0 µg/mL Streptavidin-APC
0,5 µg/mL Streptavidin-APC
5,0 µg/mL Streptavidin-APC

Fig. 6:

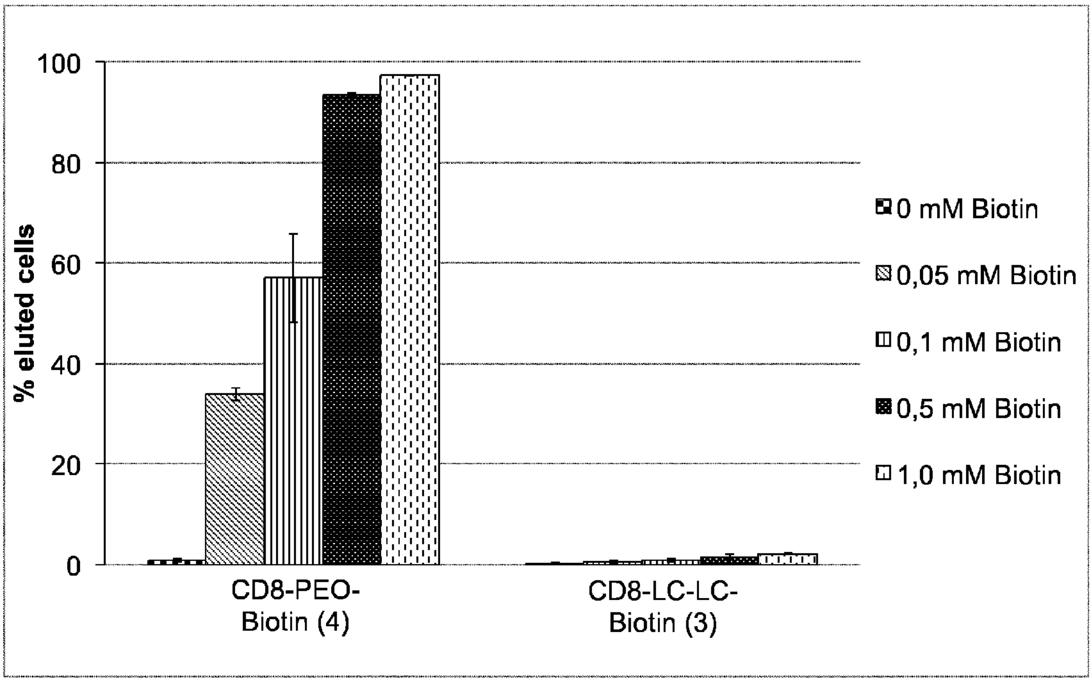
100
80
60
40
20
0
% eluted cells
0 mM Biotin
0,05 mM Biotin
0,1 mM Biotin
0,5 mM Biotin
1,0 mM Biotin
CD8-PEO-Biotin (4)
CD8-LC-LC-Biotin (3)

Fig. 7:

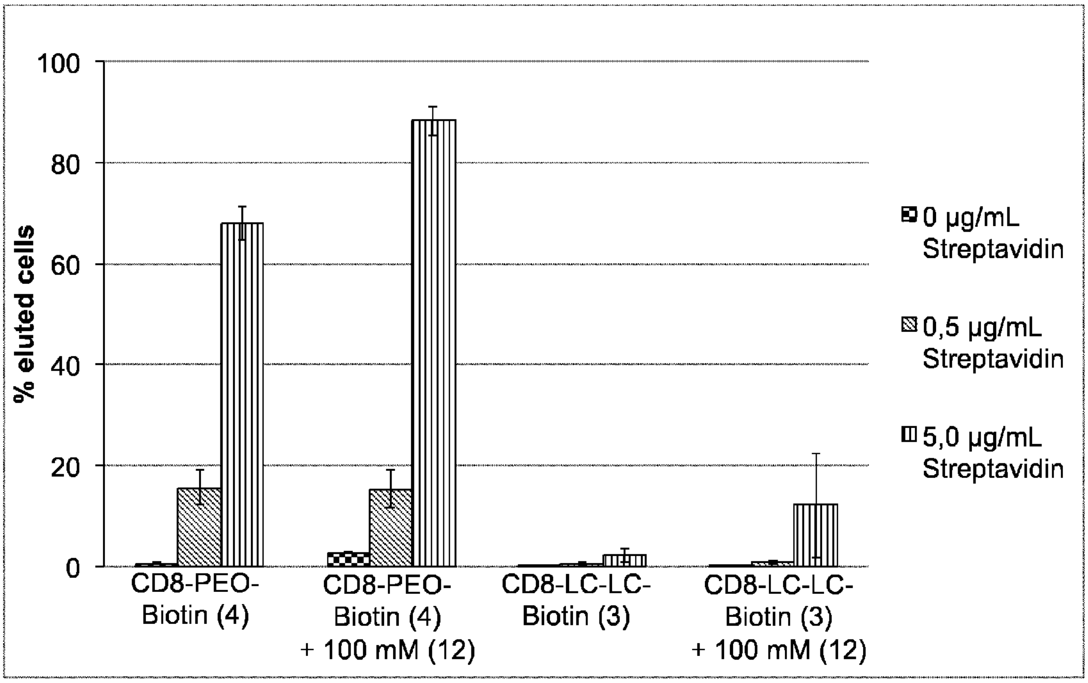
100
80
60
40
20
0
% eluted cells
0 µg/mL Streptavidin
0,5 µg/mL Streptavidin
5,0 µg/mL Streptavidin
CD8-PEO-Biotin (4)
CD8-PEO-Biotin (4) + 100 mM (12)
CD8-LC-LC-Biotin (3)
CD8-LC-LC-Biotin (3) + 100 mM (12)

Fig. 8:

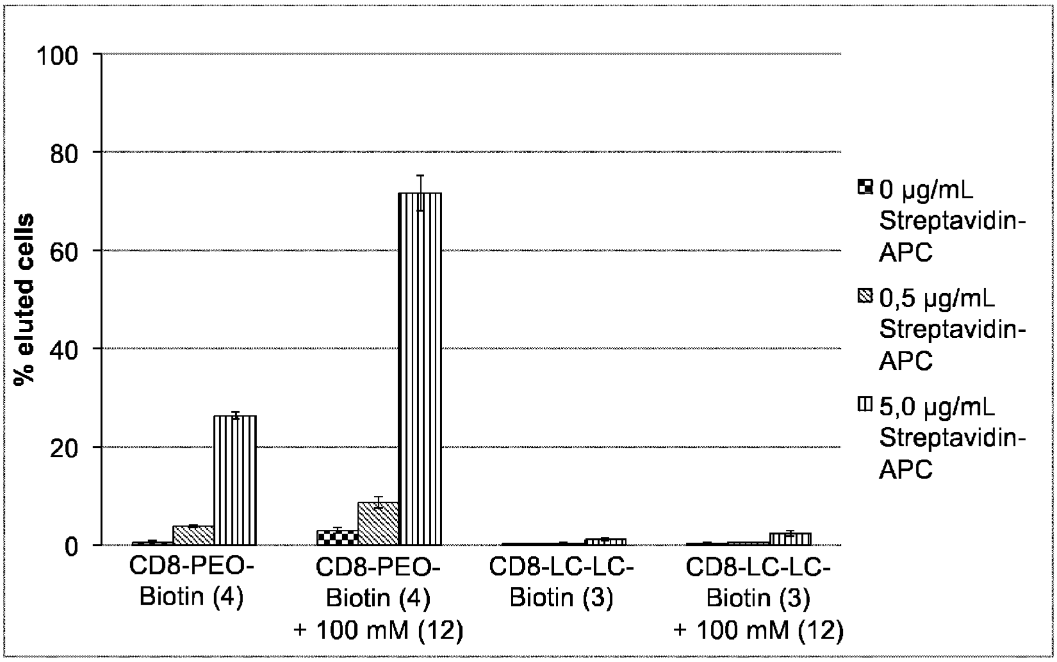
100
80
60
40
20
0
% eluted cells
CD8-PEO-Biotin (4)
CD8-PEO-Biotin (4) + 100 mM (12)
CD8-LC-LC-Biotin (3)
CD8-LC-LC-Biotin (3) + 100 mM (12)
0 µg/mL Streptavidin-APC
0,5 µg/mL Streptavidin-APC
5,0 µg/mL Streptavidin-APC

Fig. 9:

Europäisches Patentamt
European Patent Office
Office européen des brevets

# EUROPEAN SEARCH REPORT

Application Number
EP 12 18 9516

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHAN KE ET AL: "Avidin-Biotin-PEG-CPA Complexes as Potential EPR-Directed Therapeutic Protein Carriers: Preparation and Characterization", BIOCONJUGATE CHEMISTRY, vol. 18, no. 5, 1 September 2007 (2007-09-01), pages 1644-1650, XP55050030, ISSN: 1043-1802, DOI: 10.1021/bc700182t * the whole document * * figures 1, 2 * * page 1646, column 1 * ----- | 1-15 | INV. G01N33/532 G01N33/543 A61K47/48 |
| X | KE S ET AL: "Intermolecular interaction of avidin and PEGylated biotin", BIOCONJUGATE CHEMISTRY, ACS, WASHINGTON, DC, US, vol. 18, no. 6, 21 November 2007 (2007-11-21), pages 2109-2114, XP002573017, ISSN: 1043-1802, DOI: 10.1021/BC700204K [retrieved on 2007-10-19] * the whole document * * abstract * ----- -/-- | 1-15 | TECHNICAL FIELDS SEARCHED (IPC): G01N A61K |

1 The present search report has been drawn up for all claims

| Place of search | Date of completion of the search | Examiner |
|---|---|---|
| The Hague | 17 January 2013 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 18 9516

**DOCUMENTS CONSIDERED TO BE RELEVANT**

| Category | Citation of document with indication, where appropriate, of relevant passages | Relevant to claim | CLASSIFICATION OF THE APPLICATION (IPC) |
|---|---|---|---|
| X | RAMPERSAUD A A ET AL: "Novel discrete PEG-based crosslinking reagents for conjugation of antibodies and proteins to biotin, fluorochromes, enzymes and gold that eliminate aggregation, improves solubility, reduces non-specific binding and enhances low level detection limits", MOLECULAR BIOLOGY OF THE CELL, vol. 22, 2011, page 1931, XP55050022, & ANNUAL MEETING OF THE AMERICAN-SOCIETY-FOR-CELL-BIOLOGY (ASCB); DENVER, CO, USA; DECEMBER 03 -07, 2011 ISSN: 1059-1524 * the whole document * * figures 1,4 * ----- | 1-15 | |
| X | US 2007/092558 A1 (HEAVNER GEORGE [US] ET AL) 26 April 2007 (2007-04-26) * the whole document * * figure 1 * * claims 1-4,11-14 * ----- | 1-15 | |
| X | US 2003/003514 A1 (KOVALENKO VICTOR [US]) 2 January 2003 (2003-01-02) * the whole document * * figure 15 * * claims 1-6 * ----- | 1-15 | TECHNICAL FIELDS SEARCHED (IPC) |

The present search report has been drawn up for all claims

1

| Place of search | Date of completion of the search | Examiner |
|---|---|---|
| The Hague | 17 January 2013 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 18 9516

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2013

| Patent document cited in search report | Publication date | Patent family member(s) | Publication date |
|---|---|---|---|
| US 2007092558 A1 | 26-04-2007 | EP 1940444 A2 | 09-07-2008 |
| | | JP 2009512696 A | 26-03-2009 |
| | | US 2007092558 A1 | 26-04-2007 |
| | | WO 2007048121 A2 | 26-04-2007 |
| US 2003003514 A1 | 02-01-2003 | AU 2002259117 A1 | 18-11-2002 |
| | | CA 2446246 A1 | 14-11-2002 |
| | | EP 1390729 A2 | 25-02-2004 |
| | | US 2003003514 A1 | 02-01-2003 |
| | | US 2005042696 A1 | 24-02-2005 |
| | | US 2007148707 A1 | 28-06-2007 |
| | | WO 02090961 A2 | 14-11-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080255004 A
- US 5506121 A
- US 5215927 A
- WO 9216841 A
- US 5518882 A
- US 6869606 B
- US 4656252 A


### Non-patent literature cited in the description


- **M. QURESHI et al.** *J. Biol. Chem.,* 2001, vol. 276, 46422-46428
- **T. SANO et al.** *Proc. Natl. Acad. Sci. USA,* 1995, vol. 92, 3180-3184
- *Analytical Biochemistry,* 2002, vol. 308, 343-357
- **JAMES HIRSCH et al.** *Analytical Biochemistry,* 2002, vol. 308, 343-357